# EUROPÄISCHE PATENTSCHRIFT

(11) **EP 0 734 734 B1**
(45) Veröffentlichungstag und Bekanntmachung des Hinweises auf die Patenterteilung: **07.07.1999**
(21) Anmeldenummer: 95104819.8
(22) Anmeldetag: 31.03.1995
(51) Int. Cl.: A61L 11/00

(54) **Verfahren und Vorrichtung zur Desinfektion oder Sterilisation von Infektionsmüll**
Method and apparatus for desinfection or sterilisation of infectious waste
Procédé et appareil pour la désinfection ou la stérilisation de déchets infectés

(43) Veröffentlichungstag der Anmeldung: 02.10.1996
(73) Patentinhaber: Eser, Hermann, D-81927 München (DE); Kneifel, Eduard, D-82008 Unterhaching (DE)
(72) Erfinder: Eser, Hermann, D-81927 München (DE); Kneifel, Eduard, D-82008 Unterhaching (DE)
(74) Vertreter: Kohlmann, Karl Friedrich, Dipl.-Ing.

(56) Entgegenhaltungen:
- WO-A-94/00162
- WO-A-94/26320
- WO-A-95/14496
- DE-A- 4 225 430
- DE-A- 4 417 512

## Beschreibung

Die Erfindung betrifft ein Verfahren und eine Vorrichtung zur Desinfektion oder Sterilisation von Infektionsmüll. Derartiger Infektionsmüll, der zunächst desinfiziert oder sterilisiert werden muß, bevor er dann weiterbehandelt werden kann, fällt z.B. in Krankenhäusern an.

Aus der DE-A-37 10 156 ist eine Vorrichtung zur Behandlung von Infektionsmüll mit Hilfe von Mikrowellen bekannt. Diese Vorrichtung umfaßt eine Mikrowellenkammer, die auf gegenüberliegenden Seiten mit Schleusentüren versehen ist. Zwischen den beiden Schleusentüren ist ein alternierend hin- und herbewegbares Transportband oder ein Drehtisch angeordnet und darüber sind in der Kammer mehrere Mikrowellensender vorgesehen. Der zu behandelnde Infektionsmüll wird in einem verschlossenen, mikrowellendurchlässigen Behälter auf das Transportband bzw. auf den Drehtisch gestellt und anschließend mit Infrarot-Strahlung beaufschlagt, während der Behälter alternierend hin- und herbewegt bzw. alternierend um die Achse des Drehtisches gedreht wird. Durch die Bewegung des Behälters soll bewirkt werden, daß alle Bereiche des Infektionsmülls mit Mikrowellen bestrahlt werden. Ferner kann in dem Behälter ein Flüssigkeitsbeutel vorgesehen sein, der bei der Mikrowellenbestrahlung platzt, um den Infektionsmüll zu befeuchten.

Bei dieser bekannten Vorrichtung und dem zugehörigen Behandlungsverfahren ist sehr problematisch, daß unter Umständen nicht sichergestellt ist, daß der Infektionsmüll nach der Behandlung tatsächlich desinfiziert oder sterilisiert ist. Denn die Mikrowellen allein wirken nicht desinfizierend oder sterilisierend, sondern die Behandlung wird nur dadurch wirksam, daß die Mikrowellen ein Medium, wie z.B. Wasser, erhitzen und der Infektionsmüll durch dieses erhitzte Medium desinfiziert oder sterilisiert wird. Bei dieser vorbekannten Lösung besteht daher die Gefahr, daß das Einbringen des Flüssigkeitsbeutels vom Bedienpersonal vergessen wird und bei nicht ausreichend feuchtem Infektionsmüll keine Desinfektion oder Sterilisation erfolgt.

Um dieses Problem, daß das Zugeben von Flüssigkeit in den Infektionsmüllbehälter vergessen wird, zu lösen, schlägt die EP-A-0 410 306 ein Verfahren und eine Vorrichtung zur Sterilisation von Müll, insbesondere Krankenhausmüll vor, bei dem mit Infektionsmüll gefüllte Kunststoffbehälter mit Injektionsnadeln angestochen werden, um Wasser und/oder ein Desinfektionsmittel in das Innere der Kunststoffbehälter einzuspritzen.

Dazu wird eine Mehrzahl von speziellen, mit Infektionsmüll gefüllten Kunststoffbehältern auf ein Vorbereitungsförderband in einer Schleusenkammer aufgebracht. Nach dem Verschließen der Eingangstür der Schleusenkammer wird eine Schleusentür zu einer Sterilisationskammer geöffnet und die Kunststoffbehälter werden von dem Vorbereitungsförderband an ein Hauptförderband innerhalb der Sterilisationskammer übergeben. Nach dem Verschließen der Sterilisationskammer wird eine der Behälteranzahl entsprechende Anzahl beweglicher Injektionsnadeln von oben her in entsprechende Einstichstellen in den Deckeln der Kunststoffbehälter eingestochen. Nach dem Einspritzen von Wasser oder Desinfektionsmittel in die Kunststoffbehälter werden die Behälter in der Sterilisationskammer mit Mikrowellen bestrahlt, um die Sterilisation durchzuführen. Danach werden die Injektionsnadel wieder herausgezogen und die Behälter können am Austragsende des Hauptförderbandes nach dem Öffnen einer Tür entnommen werden. Zudem wird in der EP-A-0 410 306 vorgeschlagen, jeweils noch eine zweite Injektionsnadel in die Behälter einzustechen, um zur Vermeidung von Überdruck im Kunststoffbehälter überschüssige Gase abzuleiten.

Zwar löst das vorgeschlagene Verfahren das Problem, daß das Einbringen von Flüssigkeit in den mit Infektionsmüll gefüllten Kunststoffbehälter versehentlich vergessen wird, jedoch ist die bekannte Lösung sehr aufwendig hinsichtlich des Einstechmechanismus für die Injektionsnadel sowie hinsichtlich der erforderlichen exakten Positionierung der Behälter in bezug auf die Injektionsnadeln, um sicherzustellen, daß die Injektionsnadeln präzise in die vorgesehenen Einstichstellen in den Behälterdeckeln treffen. Schließlich ergibt sich das Problem, daß die Kunststoffbehälter praktisch keiner nennenswerten Druckbelastung standhalten, so daß die während der Behandlung auftretenden Drücke im Inneren des Kunststoffbehälters nicht so eingestellt werden können, wie es für eine Desinfektion oder Sterilisation von Infektionsmüll optimal wäre.

Im Bundesgesundheitsblatt 30, Nr. 8, August 1987, ist ein fraktioniertes Vakuum-Verfahren beschrieben, bei dem die Desinfektion von Gegenständen in einer vakuumdichten Desinfektionskammer durchgeführt wird. Dabei wird die Luft aus der Kammer und aus dem Desinfektionsgut durch mehrmaliges Evakuieren im Wechsel mit Einströmenlassen von Sattdampf entfernt, anschließend erfolgt die Desinfektion mit Sattdampf und schließlich wird das Desinfektionsgut durch Evakuieren der Kammer getrocknet. Derartige Verfahren werden in der Praxis mit sehr hohen Drücken und Temperaturen durchgeführt und benötigen daher aufwendige und teuere Anlagen. Der Sattdampf wird dabei extern erzeugt und dann der evakuierten Kammer zugeführt. Zwar liefert dieses vorbekannte Verfahren sehr gute Sterilisationsergebnisse, jedoch erfordert es kosteninstensive, stationäre Anlagen.

Der Erfindung liegt das Problem zugrunde, ein Verfahren und eine Vorrichtung zur Desinfektion oder Sterilisation von Infektionsmüll zu schaffen, womit eine zuverlässige Desinfektion oder Sterilisation des Infektionsmülls kostengünstig erreicht wird.

Dieses Problem wird von einem Verfahren mit den Merkmalen des Patentanspruchs 1 und von einer Vorrichtung zur Durchführung des Verfahrens mit den Merkmalen des Patentanspruchs 3 gelöst.

Beim erfindungsgemäßen Verfahren wird der zu behandelnde Infektionsmüll zunächst in eine Druckkammer eingebracht. Dann wird die Druckkammer evakuiert, um durch den Entzug von Luft und Gasen aus der Kammer und aus dem Infektionsmüll optimale Bedingungen zur Schaffung einer Atmosphäre aus gesättigtem Dampf herzustellen. Anschließend wird ein Fluid in die Druckkammer eingespeist. Dieses Fluid kann Flüssigkeit sein, die aufgrund des Vakuums im Inneren des Druckbehälters verdampft oder aber vorverdampfter Sattdampf. Die Menge des Fluides wird so gewählt, daß zumindest das Druckkammervolumen vollständig mit gesättigtem Dampf ausgefüllt wird. Der zu behandelnde Infektionsmüll verweilt dann im erhitzten gesättigten Dampf in der Druckkammer während einer vorgegebenen Verweildauer, die zur Desinfektion oder Sterilisation erforderlich ist. Schließlich wird die Druckkammer belüftet und der desinfizierte oder sterilisierte Müll kann entnommen werden.

Bei dem erfindungsgemäßen Verfahren ist eine zuverlässige Desinfektion oder Sterilisation des Infektionsmülls sichergestellt. Denn durch das Evakuieren der Druckkammer wird gewährleistet, daß nach der Einspeisung der Flüssigkeit in die Druckkammer tatsächlich nur gesättigter Dampf ohne störende Gase vorliegt, was in Verbindung mit einer entsprechenden Temperatur des gesättigten Dampfes Voraussetzung für eine ordnungsgemäße Desinfektion oder Sterilisation ist. Da die Erhitzung des Dampfes auf die zur Desinfektion oder Sterilisation erforderliche Temperatur vor dem Eintritt des Fluides in die Druckkammer erfolgt, läßt sich das erfindungsgemäße Verfahren kostengünstig realisieren.

Zwar ist aus der älteren, jedoch noch nicht vorveröffentlichten deutschen Patentschrift P 44 07 311 ein mit Vakuum und Dampf arbeitendes Verfahren zur Desinfektion und Sterilisation von Infektionsmüll bereits bekannt, jedoch wird in diesem Verfahren Mikrowellen-Strahlung zum Erhitzen des Fluides und zum Aufschmelzen einer Sollbruchstelle eingesetzt. Da bei Verfahren, die mit Mikrowellen-Strahlung arbeiten, besondere Abschirmungsmaßnahmen getroffen werden müssen, sind derartige Verfahren apparativ relativ aufwendig.

Beim erfindungsgemäßen Verfahren kann als Fluid, welches in die Druckkammer eingespeist wird, Wasser verwendet werden. Da also keine Desinfektionsmittel benötigt werden, werden Kosten gespart.

Um den Restluftgehalt des gesättigten Dampfes und die Bildung von Kondensat in der Druckkammer zu minimieren, ist es erfindungsgemäß günstig, nach dem Einspeisen von Sattdampf in die Druckkammer mehrere Zyklen durchzuführen und anschließend die Druckkammer wieder zu evakuieren. Außerdem sind die Wandungen der Druckbehälter beheizt, um ebenfalls Kondensatbildung zu verhindern.

Eine bevorzugte Ausgestaltung des erfindungsgemäßen Verfahrens bezieht sich auf die Behandlung von Infektionsmüll, der in verschlossenen Behältnissen, z.B. aus Kunststoff, vorliegt. Denn Infektionsmüll, wie er z.B. in Krankenhäusern anfällt, wird in der Regel zuerst in einem Behältnis gesammelt und anschließend in dem verschlossenem Behältnis zur Desinfektions- oder Sterilisationsbehandlung gebracht. Dabei sollte jedoch das Behältnis vor der Behandlung nicht mehr geöffnet werden, damit nicht durch austretende Keime oder Bakterien eine Kontamination der Umgebung verursacht wird. Dieses Problem, daß einerseits der Infektionsmüll im Behältnis mit heißem Sattdampf desinfiziert werden soll, andererseits aber das Behältnis außerhalb der Behandlungskammer nicht mehr geöffnet werden soll, wird erfindungsgemäß dadurch gelöst, daß nach dem Einbringen des Behältnisses mit Infektionsmüll in die Druckkammer während der sich anschließenden Evakuierung der Druckkammer gleichzeitig Infrarot-Strahlung in die Druckkammer eingebracht wird, um eine in einer Wandung des Behältnisses vorgesehene, durch Infrarot-Strahlung aufschmelzbare Sollbruchstelle zu öffnen und dadurch eine gasdurchlässige Verbindung zwischen dem Inneren des Behältnisses und der Druckkammer zu schaffen. Das Herausschmelzen der Sollbruchstelle im Behältnis wird dabei zusätzlich durch die Druckdifferenz zwischen dem Inneren des Behältnisses und der Druckkammer während der Evakuierung der Druckkammer unterstützt. Der während der Evakuierung auftretende Überdruck im Inneren des Behältnisses im Vergleich zur Druckkammer drückt also die durch Infrarot-Strahlung aufschmelzende Sollbruchstelle zusätzlich aus dem Behältnis heraus. Die Infrarot-Strahlung braucht dabei lediglich während einer Zeitdauer aufgebracht werden, die erforderlich ist, um die Sollbruchstelle sicher aufzuschmelzen. Nachdem so automatisch eine Öffnung im Behältnis hergestellt worden ist, wird dann bei der weiteren Evakuierung der Druckkammer gleichzeitig auch das Innere des Behältnisses mit dem Infektionsmüll evakuiert. Somit kann dann der erhitzte gesättigte Dampf durch die geöffnete Sollbruchstelle in das Innere des Behältnisses eindringen und die Desinfektion des Infektionsmülls bewirken.

Bei dieser Ausgestaltung des erfindungsgemäßen Verfahrens kann also der zu behandelnde Infektionsmüll in einem geschlossenen, mit einer durch Infrarot-Strahlung aufschmelzbaren Sollbruchstelle versehenen Behältnis, wie z.B. einem mit einem Deckel verschlossenen Kunststoffbehälter oder einem verschlossenen Kunststoffsack, in die Behandlungskammer eingebracht werden, ohne zuvor das Behältnis öffnen zu müssen. Während dagegen bei den eingangs beschriebenen vorbekannten Lösungen entweder die Gefahr besteht, daß vom Bedienpersonal vergessen wird, in den Sammelbehälter für den Infektionsmüll einen Flüssigkeitsbeutel einzulegen, oder aber ein sehr aufwendiger Mechanismus mit beweglichen Injektionsnadeln erforderlich ist, um dem Behälterinneren automatisch Flüssigkeit zuzuführen, wird erfindungsgemäß das Problem, den Infektionsmüll zuverlässig einer erhitzten Sattdampfatmosphäre auszusetzen, auf einfache Weise gelöst, ohne daß dabei ein manuelles oder mechanisches Eingreifen erforderlich wäre.

Dadurch, daß nach dem Durchschmelzen der Sollbruchstelle auch das Innere des Behältnisses mit dem Infektionsmüll evakuiert wird, ist zuverlässig gewährleistet, daß der Infektionsmüll tatsächlich einer reinen Sattdampfatmosphäre ausgesetzt ist. Dagegen ist bei den eingangs geschilderten Verfahren der Sammelbehälter mit dem Infektionsmüll stets mit Luft gefüllt und zudem können sich darin andere Gase gebildet haben, da man keinen Einfluß darauf hat, was im einzelnen im Behälter gesammelt worden ist. Bei den vorbekannten Verfahren ist aber eine Evakuierung des Inneren des Sammelbehälters nicht möglich, da die verwendeten Kunststoffbehälter dem dabei entstehenden Unterdruck nicht standhalten würden. Deswegen ist es bei den bekannten Verfahren auch nicht möglich, eine Sattdampfatmosphäre mit Überdruck in den Sammelbehältern für den Infektionsmüll zu schaffen, da sonst der Behälter platzen würde. Demgegenüber wirkt erfindungsgemäß ein Überdruck der Sattdampfatmosphäre lediglich auf die dafür ausgelegte Druckkammer und nicht auf das Behältnis, in dem der Infektionsmüll in die Druckkammer eingebracht wird, da durch die gasdurchlässige Verbindung zwischen dem Inneren des Behältnisses und der Druckkammer ein Druckausgleich geschaffen ist.

Zur Desinfektion von Infektionsmüll mit dem erfindungsgemäßen Verfahren ist es zweckmäßig, den gesättigten Dampf auf eine Temperatur von ≥ 105°C aufzuheizen. Für eine Sterilisation von Infektionsmüll ist dann ein geeignetes höheres Temperaturniveau als für eine Desinfektion erforderlich.

Eine besonders wirtschaftliche Ausführung des erfindungsgemäßen Verfahrens stellt die Möglichkeit dar, die Anlage, bei Vorhandensein mindestens zweier Druckbehälter im Gegentakt zu fahren. Dabei wird der eine Druckbehälter evakuiert, während der andere Druckbehälter bedampft wird.

Eine erfindungsgemäße Vorrichtung zur Durchführung des oben beschriebenen Verfahrens umfaßt einen Druckbehälter, der mit zumindest einer Zugangseinrichtung zum Einbringen und Entnehmen des Infektionsmülls und mit zumindest einer Infrarot-Strahlungsquelle versehen ist, und ein Versorgungssystem zur Evakuierung/Belüftung des Druckbehälters und zur Einspeisung von Flüssigkeit in den Druckbehälter sowie ein Steuer-, Regelungs- und Kontrollsystem.

Da beim erfindungsgemäßen Verfahren bei jedem Behandlungszyklus das gesamte Innere des Druckbehälters desinfiziert oder sterilisiert wird, können bei der erfindungsgemäßen Vorrichtung aufwendige Schleusensysteme und vorgeschaltete Schleusenkammern entfallen. Somit läßt sich die Vorrichtung zur Durchführung des erfindungsgemäßen Verfahrens vergleichsweise kompakt und mit relativ geringem konstruktiven Aufwand realisieren, was wiederum zu einer kostengünstigen Lösung führt.

Da für die Druckkammer kein Schleusensystem und auch keine Fördermittel in ihrem Inneren erforderlich sind, um, wie bei herkömmlichen Vorrichtungen, mit Infektionsmüll gefüllte Behälter hin und her zu bewegen oder zu positionieren, lassen sich aufgrund der erfindungsgemäß möglichen kompakten Bauweise der Druckbehälter in vorteilhafter Weise mehrere Druckbehälter in einer Vorrichtung integrieren. Jeder Druckbehälter ist dann mit eigenen Zugangseinrichtungen und eigenen Infrarot-Strahlungsquelle ausgestattet. Diese Druckbehälter verfügen dann günstigerweise über ein gemeinsames Versorgungssystem und ein gemeinsames Steuer-, Regelungs- und Kontrollsystem. Somit wird eine Anlage geschaffen, die sehr flexibel an den jeweiligen Bedarf an zu behandelndem Infektionsmüll angepaßt werden kann, wodurch sich der Energie- und Kostenaufwand für die Behandlung des Infektionsmülls reduziert.

Um eine kompakte und kostengünstige Vorrichtung zu erhalten, ist es vorteilhaft, den Druckbehälter in seinen Innenabmessungen nur um soviel größer als vorgegebene Maximalabmessungen für ein Infektionsmüll-Sammelbehälnis zu dimensionieren, wie es erforderlich ist, um ein Sammelbehältnis mit diesen Maximalabmessungen bequem im Druckbehälter aufnehmen zu können. Ein solcher Druckbehälter ist also nicht wesentlich größer als ein vorgegebener Sammelbehälter für Infektionsmüll. Damit kann man mit geringem Energie- und Zeitaufwand auch einen einzelnen Sammelbehälter mit Infektionsmüll behandeln und braucht nicht ein wesentlich größeres Volumen zu evakuieren, mit Sattdampf zu füllen und aufzuheizen, als es dem Volumen des Sammelbehälters mit Infektionsmüll entspricht.

Aufgrund der kompakten Abmessungen eines einzelnen Druckbehälters lassen sich dann, wie bereits zuvor beschrieben, mehrere Druckbehälter modular zu einer Anlage zusammenfassen, in der eine dem jeweiligen Bedarf entsprechende Anzahl von Infektionsmüll-Sammelbehältern gleichzeitig behandelt werden kann. Die verschiedenen Druckbehälter einer Anlage können natürlich unterschiedlich groß dimensioniert sein, jedoch ist es zweckmäßig, identische Druckbehälter einer bestimmten Standardgröße vorzusehen, um die Herstellungskosten der Anlage gering zu halten.

Vorzugsweise ist je Druckbehälter nur eine Zugangseinrichtung vorgesehen. Insbesondere ist es günstig, den Druckbehälter so zu gestalten, daß ein Teil des Druckbehälters aufklappbar ist. Auf diese Weise läßt sich der Infektionsmüll bequem in den Druckbehälter einbringen. Die Infektionsmüll-Sammelbehältnisse können nach dem Aufklappen des beweglichen Druckbehälterteils in verschlossenem Zustand direkt in den Druckbehälter hineingestellt oder -gelegt werden und nach dem Verschließen des klappbaren Druckbehälterteils kann sofort der Behandlungszyklus zur Desinfektion oder Sterilisation des Infektionsmülls begonnen werden. Die Entnahme des behandelten Infektionsmülls erfolgt dann auf dem gleichen Wege, indem das Behältnis mit dem Müll dem Druckbehälter wieder direkt entnommen wird. Somit ist eine schnelle, direkte Be- und Entladung der Druckbehälter möglich. Durch die damit erreichbare Zeiteinsparung werden die Betriebskosten gegenüber herkömmlichen Anlagen gesenkt, bei denen zum Teil beim Be- und Entladen zeitraubende Schleusensysteme passiert werden müssen.

Bei einer bevorzugten Ausführung ist jeweils nur eine Infrarot-Strahlungsquelle je Druckbehälter vorgesehen. Diese Infrarot-Strahlungsquelle kann dann optimal an die Anforderungen hinsichtlich der für einen Druckbehälter benötigten Strahlungsenergie angepaßt werden. Somit kann der konstruktive Aufwand für die erfindungsgemäße Vorrichtung verringert werden und die Vorrichtung daher kostengünstig hergestellt und wirtschaftlich betrieben werden. Wenn mehrere Druckbehälter zu einer Anlage zusammengefaßt werden, werden durch die individuell an den Druckbehältern vorgesehenen Infrarot-Strahlungsquellen gezielt jeweils nur diejenigen Druckbehälter mit Infrarot-Strahlung beaufschlagt, die auch tatsächlich mit Infektionsmüll befüllt sind. Die erfindungsgemäße Anlage aus mehreren Druckbehältern läßt sich also optimal an das zu behandelnde Infektionsmüllvolumen anpassen und es werden nicht mehr Druckbehälter als erforderlich mit Infrarot-Strahlung beaufschlagt und mit erhitztem Sattdampf gefüllt. Die erfindungsgemäße Vorrichtung arbeitet also sehr energiesparend und kostengünstig und ist sehr flexibel hinsichtlich der zu behandelnden Infektionsmüllmenge.

Die erfindungsgemäße Vorrichtung wird vorzugsweise auf ein Fahrgestell montiert und mobil betrieben, sie kann aber natürlich auch stationär betrieben werden. Dabei ist es zweckmäßig, wie bereits vorher beschrieben, mehrere Druckbehälter zusammenzufassen und mit einem gemeinsamen Versorgungssystem und einem gemeinsamen Steuer-, Regelungs- und Kontrollsystem zu versehen.

Die Form eines einzelnen Druckbehälters kann erfindungsgemäß so gewählt werden, wie es für die jeweilige Anwendung am günstigsten ist. Unter dem Gesichtspunkt einer kompakten Zusammenfassung mehrerer Druckbehälter zu einer Anlage kann beispielsweise das Kriterium ausschlaggebend sein, daß man die einzelnen Druckbehälter nebeneinander mit möglichst wenig Platzverlust anordnen kann und das zur Verfügung stehende Volumen von den Druckbehältern möglichst gut ausgenutzt wird. Insofern können beispielsweise im wesentlichen quaderförmige Druckbehälter vorteilhaft sein. Die Druckbehälter können aber auch tonnenförmig, beispielsweise mit einem Kreisquerschnitt, ausgebildet sein. Die Gestalt der Druckbehälter hängt natürlich auch von der Gestalt der Infektionsmüll-Sammelbehältnisse ab, die vom Druckbehälter zur Behandlung des Infektionsmülls aufgenommen werden sollen, und umgekehrt.

Die Infrarot-Strahlungsquellen können an den Druckbehältern in einer beliebiegen, zum aufschmelzen der Sollbruchstelle zweckmäßigen Position angeordnet werden, z.B. oben auf dem Druckbehälter seitlich oder unterhlab des Druckbehälters. Die Einstrahlung in den Druckbehälter kann dann durch ein, für die verwendete Infrarot-Strahlung durchlässiges, Fenster erfolgen.

Eine besonders vorteilhafte Ausführung der Vorrichtung besteht darin, daß Infektionsmüllbehältnisse Verwendung finden, deren Kunststoffdeckel mit einer Infrarotstrahlung absorbierenden Schicht versehen sind. Durch die Infrarotstrahlung absorbierende Beschichtung wird der Kunststoffdeckel in diesem Bereich bei Infraroteinstrahlung besonders stark erwärmt, was schließlich zum Aufschmelzen des Kunststoffdeckels führt. Das in der Druckkammer vorherrschende Druckgefälle unterstützt und beschleunigt den Öffnungsvorgang.

Eine sinnvolle Ausführung der erfindungsgemäßen Vorrichtung stellt die Verwendung eines Reflektors dar, welcher die Infrarotstrahlung so bündelt, daß der Brennpunkt unterhalb der noch zu schmelzenden Oberfläche des Kunststoffbhälters liegt. Hierdurch ist es möglich, unterschiedlich hohe Behälter aufzuschmelzen, da der Brennpunkt unterhalb der Oberfläche des niedrigsten Behälters liegt.

Die Anlage kann erfindungsgemäß modular in Zweiergruppen aufgebaut sein, wodurch unnötig große Volumina vermieden sind, was Energie und Zeit spart. Bei geringer Auslastung kann z.B. nur eine Gruppe bzw. ein Druckbehälter betrieben werden.

Nachfolgend wird ein Ausführungsbeispiel einer nach dem erfindungsgemäßen Verfahren arbeitenden Vorrichtung mit Bezug auf die beigefügten Zeichnungen näher erläutert.

Es zeigen:
- Fig. 1: eine Desinfektionsanlage gemäß der Erfindung in einer Seitenansicht;
- Fig. 2: die Anlage in Fig. 1 in der Draufsicht;
- Fig. 3: eine Prinzipskizze des Aufbaus der Anlage in Fig. 1;
- Fig. 4: ein Druckkesseloberteil mit einer Infrarot-Strahlungsquelle gemäß der Erfindung im Längsschnitt;
- Fig. 5: das Druckkesseloberteil mit Infrarot-Strahlungsquelle in Fig. 4 in der Draufsicht;
- Fig. 6: ein Schema für eine Befüllung eines Druckkessels gemäß der Erfindung mit in einem Behältnis gesammelten Infektionsmüll.

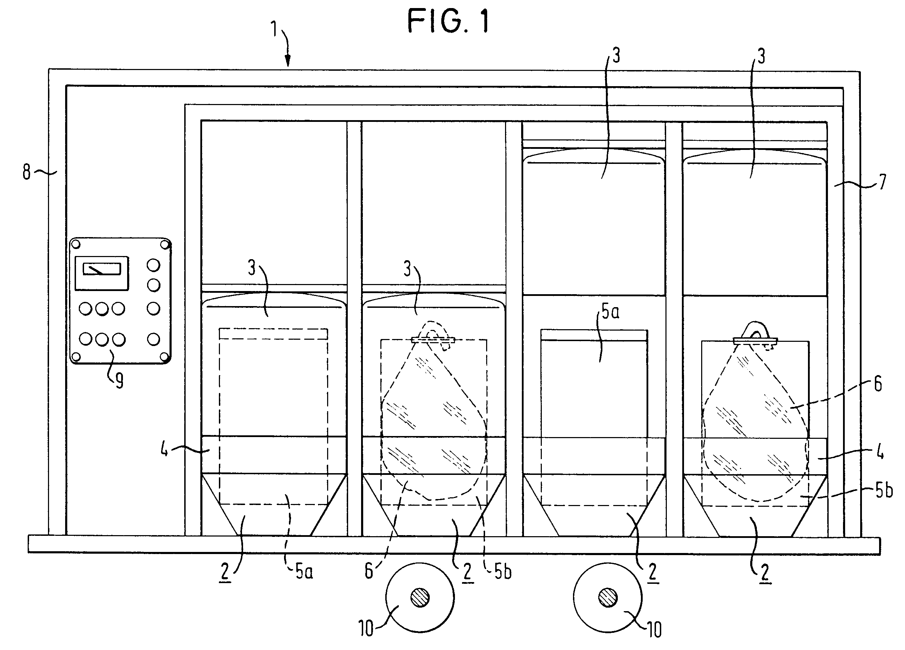

Fig. 1 zeigt eine Anlage 1 zur Desinfektion von Infektionsmüll, die vier identisch ausgeführte Druckkessel 2 umfaßt. Die vier Druckkessel 2 sind nebeneinander in einem Rahmen 7 angeordnet, der wiederum von einem Container 8 aufgenommen wird. Die gesamte Anlage im Container 8 ist auf ein - nur schematisch dargestelltes - Fahrgestell 10 montiert. Die Anlage kann beispielsweise als Aufbau auf einem Lastkraftwagen oder auf einem Anhänger montiert sein, um sie mobil betreiben zu können.

Die Druckkessel 2 bestehen jeweils aus einem aufklappbaren Oberteil 3 und einem fest montierten Unterteil 4. In Fig. 1 sind die beiden linken Druckkessel 2 in verschlossenem Zustand dargestellt, während die beiden rechten Druckkessel 2 in geöffnetem Zustand gezeigt sind. In der Abbildung sind zwei der Druckkessel 2 mit Kunststoffbehältern 5a befüllt, die jeweils mit einem Deckel verschlossen sind und in denen sich der zu behandelnde Infektionsmüll befindet. Die zwei anderen Druckkessel 2 sind jeweils mit einem offenen Kunststoffbehälter 5b und einem darin befindlichen Kunststoffmüllsack 6 befüllt. Im verschlossenen Kunststoffmüllsack befindet sich der zu behandelnde Infektionsmüll. Die vier Druckkessel 2 können also einzeln durch Öffnen der hochklappbaren Oberteile 3 mit Infektionsmüll beladen werden. Neben dem Rahmen 7 mit den vier Druckkesseln 2 ist im Container 8 ein Bedienfeld 9 zum Steuern der Desinfektionsbehandlung in den Druckkesseln vorgesehen.

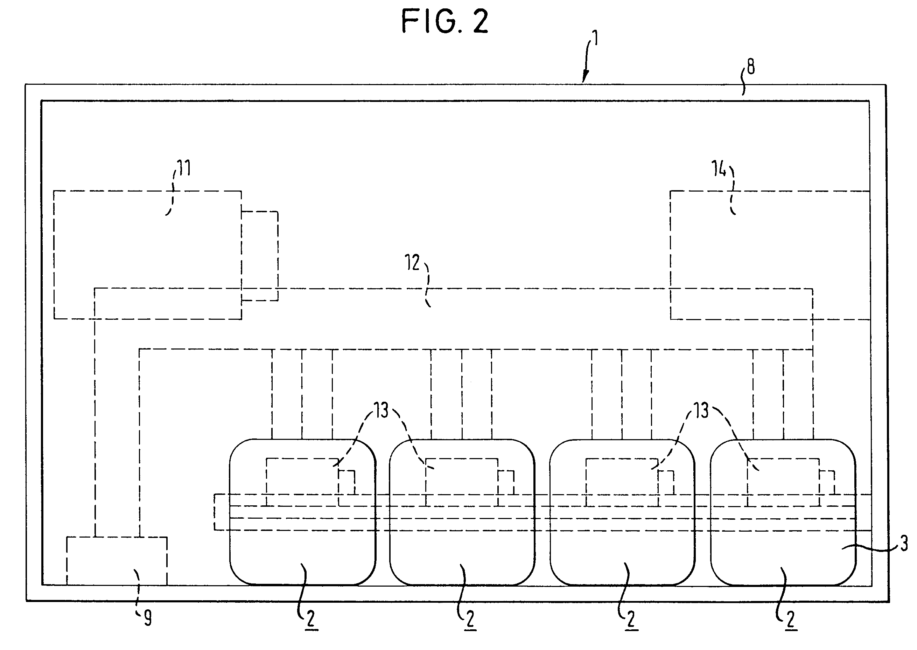

In Fig. 2 ist die Anordnung der vier Druckkessel 2 im Container 8 ersichtlich. Jeder Druckkessel 2 weist einen Öffnungsmechanismus 13 auf, mit dem das bewegliche Oberteil 3 des Druckkessels geöffnet und wieder druckdicht verschlossen werden kann.

Die vier Druckkessel 2 sind zu einer Baugruppe zusammengefaßt und verfügen über gemeinsame Steuer- und Betriebseinrichtungen. Steuer- und Versorgungsleitungen 12 sind in Fig. 2 schematisch dargestellt. Weiterhin sind eine Vakuumpumpe 11 und ein Wassertank 14 der Anlage im Container 8 dargestellt.

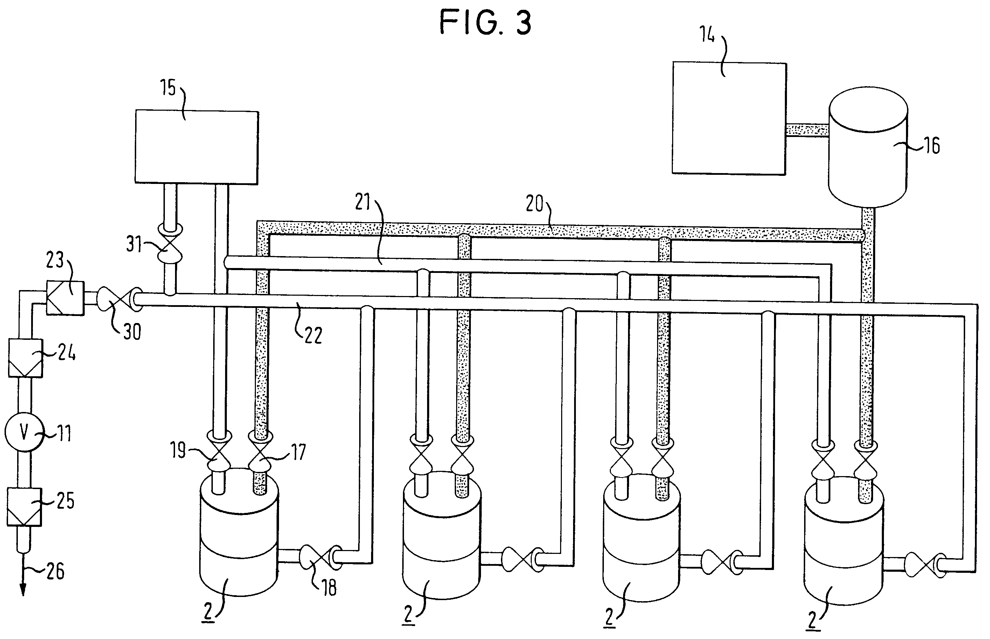

In Fig. 3 sind die Versorgungs- und Betriebssysteme der in den Figuren 1 und 2 dargestellten Anlage in ihrem schematischen Aufbau gezeigt.

Die vier Druckkessel 2 sind jeweils über ein Luftabsperrventil 18 an eine gemeinsame Be- und Entlüftungsleitung 22 angeschlossen. Die Be- und Entlüftungsleitung 22 ist über ein Absperrventil 30 mit einem Bakterienfilter 23 verbunden, der über einen Wasserabscheider verfügt. Dem ersten Bakterienfilter 23 mit Wasserabscheider ist ein zweiter Bakterienfilter 24 nachgeschaltet, an den sich die Vakuumpumpe 11 anschließt. Der Vakuumpumpe 11 ist ein Kohlefilter 25 nachgeschaltet, der in einen Luftaustritt 26 mündet.

Jeder Druckkessel 2 ist jeweils über ein Wassereinlaßventil 17 mit einer Wasserleitung 20 verbunden, die von einem Wassertank 14 gespeist wird. In der Wasserleitung 20 ist ein Boiler oder Durchlauferhitzer 16 vorgesehen, um das Speisewasser aus dem Wassertank 14 zu erhitzen, bevor es den einzelnen Druckkesseln 2 zugeführt wird.

Schließlich ist jeder Druckkessel 2 mit jeweils einem Überdruckventil 19 ausgestattet, an das sich eine Überdruckleitung 21 anschließt, die mit einem Überdruckkessel 15 verbunden ist. Der Überdruckkessel 15 wiederum ist über ein Absperrventil 31 mit der Be- und Entlüftungsleitung 22 verbunden.

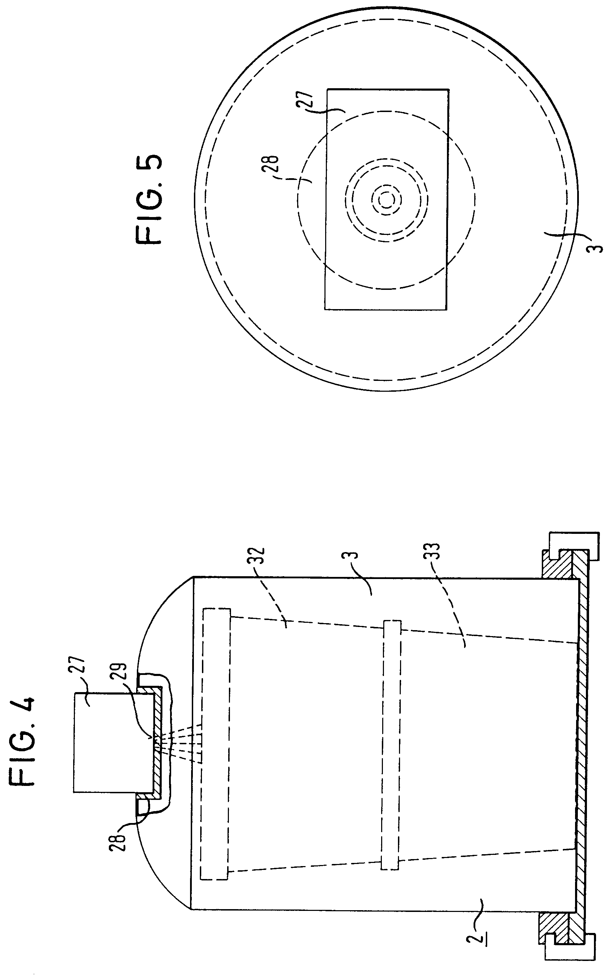

Außerdem ist jeder Druckkessel 2 mit einer Infrarot-Strahlungsquelle versehen, die jedoch in den Fig. 1 bis 3 nicht dargestellt ist. Eine solche Infrarot-Strahlungsquelle 27, wie sie an jedem Druckkessel 2 angeordnet ist, ist in Fig. 4 gezeigt. Im Ausführungsbeispiel ist eine Infrarot-Strahlungsquelle 27 außen auf der Oberseite des abhebbaren Oberteils 3 des Druckkessels 2 angeordnet, sie kann aber auch an einer anderen geeigneten Stelle angeordnet sein. Im Oberteil 3 ist eine Öffnung 29 vorgesehen, durch die die Infrarot-Strahlung in das Innere des Druckkessels 2 eingestrahlt wird. Die Öffnung 29 und ein in das Innere des Druckkessels 2 hineinragender Teil der Infrarot-Strahlungsquelle 27 ist im Inneren des Druckkessels mit einer infrarot-durchlässigen Abdeckung 28 versehen. Diese Abdeckung 28 dient dem Schutz der Infrarot-Strahlungsquelle 27 vor dem Druck, der Feuchtigkeit und der Hitze, die im Druckkessel auftreten. Fig. 4 und 5 zeigen die Anordnung der Infrarot-Strahlungsquelle 27 und der Abdeckung 28 zentral an der Oberseite des abhebbaren Oberteils 3 des Druckkessels. Außerdem sind zwei Infektionsmüllbehältnisse 32, 33 unterschiedlicher Größe angedeutet.

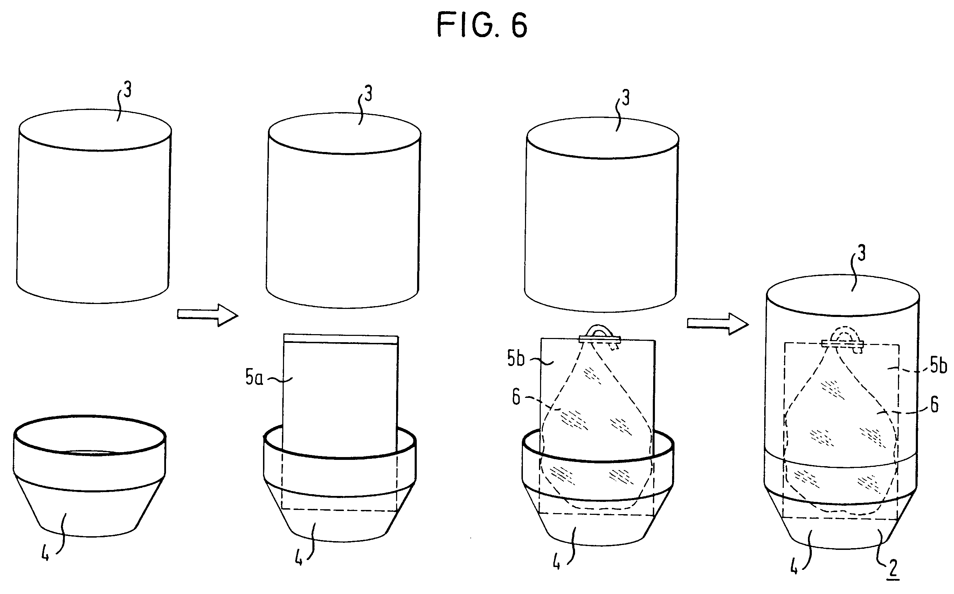

In Fig. 6 ist schematisch ersichtlich, wie ein Druckkessel 2, wie er zuvor mit Bezug auf die vorangehenden Figuren beschrieben wurde, mit Infektionsmüll befüllt wird. In der ganz linken Darstellung ist schematisch dargestellt, wie der aus einem Oberteil 3 und einem Unterteil 4 bestehende Druckkessel geöffnet wird. In der praktischen Ausführung kann dabei, wie bereits zuvor erläutert, ein Klappmechanismus zwischen den Teilen 3 und 4 ausgebildet sein oder es könnte aber auch ein Schiebemechanismus konstruiert werden, bei dem das Oberteil 3, entsprechend der Skizze in Fig. 6, vom Unterteil 4 weg nach oben geschoben wird. In den beiden mittleren Darstellungen in Fig. 6 ist alternativ die Befüllung mit einem verschlossenen Kunststoffbehälter 5a und mit einem verschlossenen Kunststoffmüllsack 6 gezeigt. Im einen Fall wird der zu behandelnde Infektionsmüll in einem stabilen Kunststoffbehälter 5a gesammelt, mit einem Deckel verschlossen und dann in verschlossenen Zustand zur Desinfektionsanlage transportiert und so in den Druckkessel eingebracht. Im anderen Fall wird der Infektionsmüll in einem Kunststoffsack 6 gesammelt, der dann verschlossen wird und so zur Desinfektionsanlage transportiert wird. Der Kunststoffsack 6 mit Infektionsmüll kann dann, wie in Fig. 6 gezeigt, in einen offenen Kunststoffbehälter 5b gelegt werden, der in den Druckkessel 2 eingebracht wird. Sowohl der mit einem Deckel verschlossene Kunststoffbehälter 5a als auch der verschlossene Kunststoffsack 6 bleiben nach dem Einbringen in den Druckkessel 2 verschlossen. Schließlich wird, wie ganz rechts in Fig. 6 dargestellt, der mit Infektionsmüll beladene Druckkessel 2 durch Zusammenklappen oder Zusammenschieben der beiden Druckkesselteile 3 und 4 druckdicht verschlossen.

Wie nachfolgend bei der Beschreibung des zugehörigen Desinfektionsverfahrens noch erläutert wird, weisen die mit Infektionsmüll gefüllten Kunststoffbehälter 5a und Kunststoffsäcke 6 jeweils eine Infrarot-Licht absorbierende Beschichtung auf.

Im folgenden wird nun beispielhaft das erfindungsgemäße Desinfektionsverfahren erläutert, wie es mit der vorangehend geschilderten Anlage durchgeführt werden kann.

Zunächst werden die Druckkessel 2, wie zuvor beschrieben, mit Infektionsmüll gefüllt, der sich entweder in verschlossenen Kunststoffbehältern 5a oder verschlossenen Kunststoffsäcken 6 befindet. Nach dem Füllen der einzelnen Druckkessel 2 werden diese druckdicht verschlossen und das Desinfektionsprogramm wird gestartet.

Dabei werden zunächst die Druckkessel 2 mittels der Vakuumpumpe 11 evakuiert, wobei die Abluft über die Bakterienfilter 23, 24 und den Kohlefilter 25 geleitet wird. Während des Evakuierens wird der Druckkesselinnenraum kurzzeitig von den Infrarot-Strahlungsquellen 27 mit Infrarot-Licht bestrahlt. Dadurch erhitzt sich der mit der zuvor erwähnten Infrarot-Licht absorbierende Beschichtung versehene Kunststoffdeckel, so daß der Kunststoff im Nahbereich der Beschichtung schmilzt und eine Öffnung im Behälter bzw. Sack entsteht. Das Öffnen der Sollbruchstelle in Form der Ausschmelzung wird durch den Überdruck im Inneren des Behälters bzw. Sacks im Verhältnis zum in der Evakuierung befindlichen Druckkesselinnenraum unterstützt. Aufgrund dieser im Kunststoffbehälter 5a bzw. Kunststoffsack 6 gebildeten Öffnung werden bei der weiteren Evakuierung sowohl dem Druckkessel selbst als auch dem Inneren des Behälters bzw. des Sacks die Luft sowie Gase entzogen.

Nach dem Erreichen eines unteren Betriebsdrucks von ca. 40mb wird der Druckkessel 2 durch das Luftabsperrventil 18 luftdicht abgesperrt und danach wird vom Boiler oder Durchlauferhitzer 16 erhitztes Wasser aus dem Wassertank 14 über die Wasserleitung 20 und das Wassereinlaßventil 17 in den Druckkessel eingespeist. Die eingespritzte Wassermenge ist etwas höher gewählt, als es für das völlige Ausfüllen des Druckkessels mit gesättigtem Wasserdampf erforderlich wäre. Durch den niedrigen Luftdruck im Druckkessel verdampft das eingespritzte Wasser. Der Wasserdampf sollte zur Desinfektion auf mindestens 105°C erhitzt sein.

Nun wird das Luftabsperrventil 18 geöffnet und der erhitzte Wasserdampf dehnt sich in der Be- und Entlüftungsleitung 22 bis zu den Absperrventilen 30 und 31 aus, die vor dem Bakterienfilter 23 bzw. dem Überdruckkessel 15 angeordnet sind. Dadurch wird sichergestellt, daß auch die Be- und Entlüftungsleitung 22 desinfiziert wird, durch die vorher die evakuierte Luft aus dem Druckkessel und dem mit Infektionsmüll gefüllten Behältnis geleitet worden ist. Andernfalls würden beim späteren Belüften des Druckkessels eventuell in den Evakuierungsgasen enthaltene Bakterien oder Erreger nach der Desinfektionsbehandlung wieder in den Druckkessel eingeleitet werden.

Dann beginnt eine ca. 3-minütige Verweilzeit, um eine gleichmäßige Erwärmung des Füllguts des Druckkessels zu gewährleisten. Daran schließt sich eine ca. 5-minütige Desinfektionszeit an, während der das zu desinfizierende Füllgut sowie die Be- und Entlüftungsleitungen desinfiziert werden.

Nach Ablauf der Desinfektionszeit wird der Druckkessel belüftet und kann dann geöffnet werden, um den desinfizierten Müll zu entnehmen.

Sollte sich einmal im zu behandelnden Infektionsmüll ein Gegenstand befinden, der während der Behandlung platzt und einen Überdruck im Druckkessel erzeugt, spricht das Überdruckventil 19 an, und das unter Überdruck stehende Gas wird in den Überdruckkessel 15 abgeleitet.

Die Druckkessel 2 sind im Ausführungsbeispiel so dimensioniert, daß sie Kunststoffbehälter oder Kunststoffsäcke, die jeweils zumindest eine mit Infrarot-Licht aufschmelzbare Sollbruchstelle aufweisen, bis zu einem Volumen von 60 Litern aufnehmen kann. Wie aus der wenn auch nur schematischen Darstellung in Fig. 1 ersichtlich ist, sind die Abmessungen der Druckkessel nur unwesentlich größer gewählt als die Abmessungen der aufzunehmenden Kunststoffbehältnisse. Im Druckkessel selbst befinden sich noch Temperaturfühler und Druckmeßwertgeber zur Steuerung und Kontrolle des Desinfektionsvorgangs.

Da bei dem vorangehend geschilderten Verfahren die Kunststoffbehälter nur thermisch und nicht mechanisch belastet werden, können diese nach entsprechender Reinigung wieder verwendet werden, wenn die thermisch auslösbare Sollbruchstelle z.B. im Behälterdeckel angeordnet ist, so daß nur der Deckel ausgetauscht werden muß. Die Wiederverwendbarkeit der Kunststoffbehälter führt auch zu einem kostengünstigen Betrieb bei der Desinfektion von Infektionsmüll. Außerdem läßt sich bei den Kunststoffbehältern Material durch Verwendung dünnerer Wandstärken einsparen, da die Behälter selbst nicht durch Über- oder Unterdruck mechanisch beansprucht werden.

Ein wesentlicher Vorteil beim erfindungsgemäßen Verfahren ergibt sich durch die Zeiteinsparung für einen vollen Desinfektionsvorgang. Da das erhitzte Wasser bei 40mb bereits verdampft, muß die Energie für die Verdampfungswärme nicht mehr zusätzlich aufgebracht werden, was Zeit und Energie spart. Während bei herkömmlichen Verfahren ein voller Behandlungszyklus in der Regel zwischen einer und mehreren Stunden dauert, werden für den vorangehend geschilderten Desinfektionszyklus lediglich etwa 20min. benötigt.

Mit dem erfindungsgemäßen Verfahren und der erfindungsgemäßen Vorrichtung zur Durchführung des Verfahrens kann also Infektionsmüll kostengünstig, schnell und in unterschiedlichen Mengen behandelt werden.

## Patentansprüche

1. Verfahren zur Desinfektion oder Sterilisation von Infektionsmüll, das folgende Schritte umfaßt:
- Einbringen des Infektionsmülls in eine Druckkammer;
- Evakuieren der Druckkammer;
- Einspeisen eines erhitzten Fluides, dessen Menge zumindest zur Füllung des Druckkammervolumens mit Sattdampf ausreicht und dessen Temperatur der gewünschten Desinfektions- oder Sterilisationstemperatur entspricht, in die Druckkammer;
- Verweilenlassen der erhitzten Sattdampffüllung in der Druckkammer während einer zur Desinfektion oder Sterilisation vorgesehenen Verweilzeit;
- Belüften der Druckkammer und Entnehmen des desinfizierten oder sterilisierten Mülls,
**dadurch gekennzeichnet,** daß während der Evakuierung der Druckkammer nach dem Einbringen des Infektionsmülls Infrarot-Strahlung in die Druckkammer eingebracht wird, um eine durch Infrarot-Strahlung aufschmelzbare Sollbruchstelle in einer Wandung eines verschlossenen Behältnisses (5a; 6), in dem sich der Infektionsmüll befindet, zu öffnen und so eine gasdurchlässige Verbindung zwischen dem Inneren des Behältnisses und der Druckkammer herzustellen.

2. Verfahren nach Anspruch 1, mit mindestens zwei Druckkammern **dadurch gekennzeichnet,** daß
das Verfahren im Gegentakt betrieben wird.

3. Vorrichtung zur Durchführung des Verfahrens nach Anspruch 1 und 2,
- mit einem Druckbehälter (2), der mit zumindest einer Zugangseinrichtung (3) zum Einbringen und Entnehmen des Infektionsmüll versehen ist, und
- einem Versorgungssystem zur Evakuierung und Belüftung des Druckbehälters und zur Einspeisung von Fluid in den Druckbehälter, sowie
- einem Steuer-, Regelungs- und Kontrollsystem
**dadurch gekennzeichnet, daß** der Druckbehälter (2) mit zumindest einer Infrarot-Strahlungsquelle (27) ausgestattet ist.

4. Vorrichtung nach Anspruch 3, **dadurch gekennzeichnet,** daß die Vorrichtung mehrere Druckbehälter (2) umfaßt, die über ein gemeinsames Versorgungssystem und ein gemeinsames Steuer-, Regelungs- und Kontrollsystem verfügen.

5. Vorrichtung nach Anspruch 3 oder 4, **dadurch gekennzeichnet,** daß der Druckbehälter (2) in seinen Innenabmessungen nur um soviel größer als vorgegebene Maximalabmessungen für ein Infektionsmüllbehältnis (5a) dimensioniert ist, wie es zur bequemen Aufnahme eines Infektionsmüllbehältnisses (5a) mit diesen Maximalabmessungen erforderlich ist.

6. Vorrichtung nach einem der Ansprüche 3 bis 5, **dadurch gekennzeichnet,** daß nur eine Zugangseinrichtung (3) für den Druckbehälter (2) vorgesehen ist.

7. Vorrichtung nach Anspruch 6, **dadurch gekennzeichnet,** daß ein Teil (3) des Druckbehälters (2) aufklappbar ist.

8. Vorrichtung nach einem der Ansprüche 3 bis 7, **dadurch gekennzeichnet,** daß nur eine Infrarot-Strahlungsquelle (27) für den Druckbehälter (2) vorgesehen ist.

9. Vorrichtung nach einem der Ansprüche 3 bis 8, **dadurch gekennzeichnet, daß** die Druckbehälter beheizt sind.

10. Vorrichtung nach Anspruch 5, **dadurch gekennzeichnet,** daß das Infektionsmüllbehältnis einen Kunststoffdeckel mit einer Infrarotstrahlung absorbierenden Beschichtung aufweist.

11. Vorrichtung nach Anspruch 3**,dadurch gekennzeichnet,** daß ein Reflektor in der Druckkammer angebracht ist, der die durch ein Einkopplunsfenster am obersten Punkt der Druckkammer aufgebrachte Infrarotstrahlung bündelt.

12. Vorrichtung nach einem der Ansprüche 3 bis 11,**dadurch gekennzeichnet,** daß die vorrichtung modular in Zweiergruppen in einer Arlage aufgebaut ist.

## Claims

1. A method for disinfecting or sterilizing infectious waste, comprising the steps of:
- introducing said infectious waste into a pressure chamber;
- evacuating said pressure chamber;
- feeding a heated fluid, the quantity of which is at least sufficient to fill the pressure chamber's volume with saturated steam and the temperature of which corresponds to the desired disinfection or sterilization temperature, into said pressure chamber;
- allowing said heated saturated steam in said pressure chamber to remain for a dwell time provided for the purpose of disinfection or sterilization;
- aerating said pressure chamber and removing said disinfected or sterilized waste,
**characterized in** that during the evacuation of said pressure chamber after said infectious waste has been introduced, infrared radiation is introduced into said pressure chamber so as to open a reference rupture point which can be melted open by infrared radiation and located in a wall of a sealed container (5a; 6) in which said infectious waste is located, thus producing a gas-permeable connection between the inside of said container and said pressure chamber.

2. A method according to claim 1 having at least two pressure chambers, **characterized in** that said method is operated in a push-pull mode.

3. An apparatus for performing said method according to claims 1 or 2,
- having a pressure vessel (2) fitted with at least one access means (3) for introducing and removing said infectious waste, and
- a supply system for evacuating and aerating said pressure vessel and for feeding fluid into said pressure vessel, as well as
- a control, regulating and monitoring system
**characterized in** that said pressure vessel (2) is fitted with at least one infrared radiation source (27).

4. An apparatus according to claim 3, **characterized in** that said apparatus comprises a plurality of pressure vessels (2) which have a joint supply system and a joint control, regulating and monitoring system.

5. An apparatus according to claims 3 or 4, **characterized in** that the internal dimensions of said pressure vessel (2) are dimensioned to be greater than predetermined maximum dimensions for an infectious-waste container (5a) only by as much as is necessary for comfortably receiving an infectious-waste container (5a) with these maximum dimensions.

6. An apparatus according to one of the claims 3 to 5, **characterized in** that just one access means (3) is provided for said pressure vessel (2).

7. An apparatus according to claim 6, **characterized in** that a part (3) of said pressure vessel (2) can be opened.

8. An apparatus according to one of the claims 3 to 7, **characterized in** that just one infrared radiation source (27) is provided for said pressure vessel (2).

9. An apparatus according to one of the claims 3 to 8, **characterized in** that said pressure vessels are heated.

10. An apparatus according to claim 5, **characterized in** that said infectious-waste container has a plastic lid with a coating which absorbs infrared radiation.

11. An apparatus according to claim 3, **characterized in** that a reflector is fitted in said pressure chamber, said reflector focusing said infrared radiation applied through a bunching window at the upper-most point of said pressure chamber.

12. An apparatus according to one of the claims 3 to 11, **characterized in** that said apparatus is composed modular in groups of two in an installation.

## Revendications

1. Procédé de désinfection ou de stérilisation de déchets infectés, comprenant les étapes suivantes :
- introduction des déchets infectés dans une chambre à pression;
- mise sous vide de la chambre à pression;
- injection dans la chambre à pression d'un fluide chauffé, dont la quantité suffit au moins à remplir au moins en vapeur saturée le volume de la chambre à pression, et dont la température correspond à la température de désinfection ou de stérilisation souhaitée;
- laisser s'écouler un temps de séjour pour le remplissage en vapeur saturée chauffée, dans la chambre à pression, pendant lequel s'écoule le temps de séjour prévu pour la désinfection ou la stérilisation;
- aérer la chambre à pression et prélever les déchets désinfectés ou stérilisés,
caractérisé en ce que, pendant la mise sous vide de la chambre à pression, après introduction des déchets infectés, est introduit dans la chambre à pression un rayonnement infrarouge pour ouvrir un point destiné à la rupture, pouvant être ouvert par fusion par l'effet d'un rayonnement infrarouge, dans une paroi d'un récipient (5a; 6) fermé, dans lequel se trouvent les déchets infectés, et établir ainsi une liaison perméable aux gaz entre l'intérieur du récipient et la chambre à pression.

2. Procédé selon la revendication 1, avec au moins deux chambres à pression, caractérisé en ce que le procédé est conduit en opposition de phases de déroulement.

3. Dispositif de mise en oeuvre selon les revendications 1 et 2,
- avec un récipient à pression (2), doté d'au moins un dispositif d'accès (3) pour introduire et prélever des déchets infectés, et
- un système d'alimentation pour évacuer et aérer le récipient à pression et pour injecter du fluide dans le récipient à pression, ainsi qu'
- un système, de commande de régulation et de contrôle,
caractérisé en ce que le récipient à pression (2) est équipé d'au moins une source de rayonnement à infrarouge (27).

4. Dispositif selon la revendication 3, caractérisé en ce que le dispositif comprend plusieurs récipients à pression (2) qui disposent d'un système d'alimentation commun et d'un système de commande, de régulation et de contrôle commun.

5. Dispositif selon la revendication 3 ou 4, caractérisé en ce que les dimensions intérieures du récipient à pression (2) sont supérieures aux dimensions maximales prédéterminées, pour un récipient à déchets infectés (5a), de la valeur nécessaire pour recevoir commodément un récipient à déchets infectés (Sa) ayant ces dimensions maximales.

6. Dispositif selon l'une des revendications 3 à 5, caractérisé en ce que n'est prévu, pour le récipient à pression (2), qu'un seul dispositif d'accès (3).

7. Dispositif selon la revendication 6, caractérisé en ce qu'une partie (3) du récipient à pression (2) est susceptible d'être ouverte par rabattement.

8. Dispositif selon l'une des revendications 3 à 7, caractérisé en ce que n'est prévu, pour le récipient à pression (2), qu'une seule source de rayonnement à infrarouge (27).

9. Dispositif selon l'une des revendications 3 à 8, caractérisé en ce que les récipients à pression sont chauffés.

10. Dispositif selon la revendication 5, caractérisé en ce que le récipient à déchets infectés présente un couvercle en matière plastique ayant un revêtement absorbant les infrarouges.

11. Dispositif selon la revendication 3, caractérisé en ce qu'est monté dans la chambre à pression un réflecteur qui focalise le rayonnement infrarouge appliqué, en passant par une fenêtre de couplage placée au point le plus haut de la chambre à pression.

12. Dispositif selon l'une des revendications 3 à 11, caractérisé en ce que le dispositif est de constitution modulaire, en groupes de deux, dans une installation.
